# EUROPEAN PATENT APPLICATION

(11) **EP 3 828 470 A1**
(43) Date of publication of application: **02.06.2021**
(21) Application number: 19383056.9
(22) Date of filing: 28.11.2019
(51) Int. Cl.: F24F 3/16, F24F 13/08

(54) **BREATHING TREATMENT EQUIPMENT**

(71) Applicant: Biowair Total Systems SL, 33211 Gijón OVIEDO (Asturias) (ES)
(72) Inventor: LLANA GARCÍA, Pedro, Luis, 33211 GIJON (ASTURIAS) (ES)
(74) Representative: Herrero & Asociados, S.L.

(57) **Abstract**

The invention relates to a breathing treatment device (100) for therapeutic purposes for placement thereof in a patient resting enclosure, the device comprising a motor-powered air recirculation system, a set of air purification filters (121, 122, 123, 124), an ultraviolet ray sterilizer (126) and a plasma electrostimulator(127), the device characterized in that it comprises laminar air flow ejection means (140), laminar air flow redirecting means (140a) redirecting flow to the face of the patient, a stabilizer (110) and anchoring means to anchor the device (100) to a wall and at a height between 100 and 130 cm with respect to the floor of the enclosure.

## Description

### Object of the invention

The object of the present invention is to provide a breathing treatment device or equipment for therapeutic purposes to be placed in the user resting enclosure, as well as a method for the treatment of breathing conditions by means of the use of said device.

### Background of the invention

A number of studies demonstrate that longevity and health are associated with the length of the sequence of telomeres at the end of cellular DNA. The shorter it is, the more possibilities there are of problems in DNA replicates and therefore cell death or failure, which is directly associated with old age and health. The length of the sequence of telomeres is affected by three factors:
The hereditary (genetic) factor, the food (diet) factor and the environmental factor (pollution, viruses and bacteria, stress, rest). Therefore, there is a need for devices that can directly affect the environmental factor, that can prevent environmental pollutants, remove nanoparticles, viruses and bacteria, and which allow the body to naturally drain out contaminants, facilitating rest, increasing sleeping time and quality, and reducing stress. It facilitates oxygenating blood and cleaning the circulatory system.

Therefore, a system is desired which does all that mentioned above, particularly cleaning and better oxygenating the blood by treating the patient while he/she sleeps. The present invention meets this demand.

### Description of the invention

The present invention relates to breathing treatment equipment which facilitates cleaning and better oxygenating the blood, particularly while the user or patient sleeps. This is achieved by air treatment 24 hours a day, 365 days a year, of the room in which the user sleeps and/or in which the user spends the most time with the breathing treatment equipment according to the present invention:
The number of nanoparticles in blood is lowered by means of the breathing treatment equipment, also preventing the entry of allergens associated with said nanoparticles and the settling of particles in blood vessels, and preventing nanoparticles from occupying the position of oxygen molecules in hemoglobin connections. Therefore, the use is primarily to improve the circulatory system, whereas purifiers are designed especially for isolated use which relieves allergy or breathing problems. The use is more preventive, continuous and long-term, for improvement of the entire body.

### Main features and main advantages of the air purification equipment according to the present invention.

### Filter system:

The breathing treatment equipment according to the present invention by means of its filter system removes viruses, bacteria, molds, yeasts, gases, volatile organic compounds, formaldehydes and any type of contaminants that may potentially reach the blood of a user through the user's breathing, obtaining a permanently and completely clean space. The user therefore benefits from ultra-healthy intensive breathing, particularly while he/she sleeps, as a result of the present invention, since the air purification equipment allows cleaning not only allergens, dust, mites, etc., from the air, but nanoparticles as well.

### Operating modes:

The breathing treatment equipment according to the present invention has two overnight operating options:
1) a first mode in which the breathing treatment equipment is 100 % silent, or
2) a second mode in which the breathing treatment equipment generates low-intensity white noise (three different levels) which, along with the permanently treated air, advantageously means that the user enjoys a deep and restorative sleep, increasing hours of rest, facilitating the nasal breathing, eliminating dryness, snoring, and in many cases preventing the use of sleep apnea masks. It has a function specifically defined to prevent insomnia. Therefore, as a result of the breathing treatment equipment according to the present invention, complete rest is obtained while the user sleeps.

### Cold or hot air jet:

The breathing treatment equipment according to the present invention allows sending to the patient immediately and in a targeted manner cool air or hot air, preventing colds since this is done with completely sterilized air, thus obtaining a thermal conditioning of the room without viruses or bacteria. It is ideal for sick people and people with immunodeficiencies. This reduces the need to open windows (contamination), using air conditioning, or using heaters which move around temperature-activated viruses and bacteria. To prevent insomnia, the thermal sensation must be kept between 18 and 24 °C.

### Removal of allergens:

The breathing treatment equipment removes allergens on both a macro level and on a level of transfer by association with nanoparticles, which is the cause of the increase in number and seriousness of allergies. Said removal must be carried out permanently, so the machine works 24 hours a day; this means that the body gradually decreases allergen levels in the entire body. This allows the patient to become more resistant to any type of allergies. In this way they no longer suffer respiratory attacks which make it difficult to oxygenate the blood and increase the heart rate. With improved blood oxygenation due to fewer respiratory attacks, the entire body will be more active, everything will work better, particularly those systems most related to microcirculation, such as the brain, the skin, the reproductive system, the immune system, etc.

Therefore, all this assures that, particularly while sleeping, the user breathes completely treated and sterilized air, allowing the body of the user to regenerate as it is free of contaminants, since it has been demonstrated that the environment inside the home is up to eight times more contaminated than outside the home and comprises that detach from the walls, ceilings, fabrics, paints, varnishes, furniture, etc., create various irritations in the respiratory system which entail inflammations which complicate breathing, creating rhinitis, snoring, apneas, and in summary, a lack of rest. Furthermore, these contaminants are not filtered out by the lungs and go directly to the blood, transporting by contact any type of allergens to all the organs through the blood.

The breathing treatment equipment according to the present invention reverses these effects of contaminants, assuring every night that the body regenerates, is purified, increasing blood oxygenation upon releasing hemoglobin from nanoparticles that reach the blood and occupy the position of oxygen molecules, preventing the transport of oxygen to the organs. This will mean that the entire body works better, particularly the purifying and immune system and microcirculation (all the internal organs internal, particularly the brain, the reproductive system, immune system, the skin, etc.). Furthermore, nanoparticles deposited in veins and arteries will be removed, since the entry of contaminants will be stopped and natural draining of the body will be facilitated while resting. The body will also enjoy the improvements throughout the rest of the day as a result of the better oxygen delivery to all the internal systems.

The size of the materials that are removed from the air can comprise up to a millimeter divided by 10,000, so it is not filtered by the lungs and directly reaches the blood. Therefore, the air purification equipment according to the present invention comprises a permanent air recirculation system of up to 3 cycles per hour/24 hours a day of air of the room that is treated by filtration equipment comprising:

### Main parts of the breathing treatment equipment according to the present invention:

The air breathing treatment equipment comprises a motor which comprises a system of tubular blades and a tubular steel integral recirculation system which allows the passage of air repeatedly through:
- A filter system or package comprising a washable pre-filter for coarse particles, a special blue antibacterial filter to keep the filter free of biological material, a HEPA 13 (High Efficiency Particulate Air) filter, and an activated carbon filter with catalytic platinum, which removes gases, formaldehydes, smells, etc.
- Furthermore, the equipment comprises a stainless steel particle filter which allows obtaining a coarse filtration during extraction of the filter system when washing said filter system or during a considerable increase in air flow rate.
- An ultraviolet ray sterilizer, breaking down the DNA of viruses and bacteria. The exposure of air to ultraviolet treatment by means of the ultraviolet ray sterilizer must be controlled certain hours a day, avoiding use thereof overnight so as not to be a nuisance for anyone who is sleeping or not to excessively sterilize the room.
- A plasma electrostimulator changing the electric charge of the contaminating nanoparticles (which are virtually impossible to filter), such that they return to the environment with the opposite charge as the air nanoparticles in the room, thus associating with the air nanoparticles because of their difference in electric charge and creating a larger particle that can indeed be filtered. The plasma electrostimulator must operate at calibrated intervals with air renewal so as not to change the charge of the entire room. These systems may be regulated by remote control from any mobile, will provide a log of the hours of use of each part of the equipment, and will allow the doctor or specialist to adjust the use to each patient remotely based on the log.
- A deflector for the continuous laminar air flow which allows directing the laminar flow towards the face of the patient for optimal breathing.

### Nanoparticle control system and laser measuring device:

The breathing treatment equipment according to the present invention can operate in the room 24 hours a day and is beneficial for health, particularly while the user sleeps. This equipment must include control systems since each user has different behaviors and tolerances. As a result, the air purification equipment comprises remote control means. All the controls for the treatment of that person may be customized.

The equipment will incorporate a standard operating system that is completely automatic and comes with a standard pre-programming and incorporates a standard use of filtration, treatment speed, and programming of the UV ray sterilizers and plasma electrostimulators such that the equipment is set only to the customs of the user. To that end, the equipment incorporates a multilevel air nanoparticle laser measuring device which measures three different values, nanoparticles of 1 micron, PM 2.5 and average PM from 2.5 to 10 microns. The combination of values of this type allows an automatic setting that is entirely based on the patient's BPR (Blood Pollution Risk), is a permanent indicator that the user can see and has extremely precise values that allow being set to each home, each patient, and each lifestyle. To do all this, the equipment will also incorporate a system which detects when the patient goes to sleep (light-sensitive cell), such that the equipment transitions at that time to night mode, assuring its operation in absolute silence. Thus the patient can sleep without any type of nuisance, without any type of apnea mask, without any type of vibration, or noise, or light or nuisance of any type.

This control system also allows family members to have real information about the use or customs, sleeping hours, etc., of the user, if this is what the user wants.

### Motor and silicon stabilizer:

Achieving the equipment working while the user sleeps without bothering said user, removing nanoparticles, is extremely difficult, since air must be passed at a high pressure but low speed through high-density HEPA air purification equipment and activated carbon air purification equipment which make noise as the air passes therethrough. To that end, another novelty will be used, said novelty being very special high-powered low-consumption motors working at low speeds and high pressures. This results in vibrations, and therefore nuisances. To that end, a unique vibration absorption system has been developed a unique vibration absorption system a high-density silicon stabilizer which, placed on the equipment, absorbs vibrations of this type, along with the body of the equipment which, for that purpose, has been made 100 % from steel and silicon, thus creating a high-density module that eliminates vibrations and allows giving the equipment a service life of over 35 years. The unit of the present invention will accompany the user 24 hours, 365 days a year, for up to 50 years.

To act on the exogenous factors affecting the length of telomeres, the quality of sleep must be an indispensable part of the treatment. Better rest and suitable oxygenation will create the ideal state to avoid stress and make the body work better; the circulation, brain, breathing, musculature, the skin, the hair, and all the internal organs in general will optimally function. As a result, to achieve 100 % silent equipment which at the same time removes nanoparticles and prevents noises, a system combining the motor and a tubular steel integral circulation system which allows continuous, uninterrupted use for 50 years has been designed.

The motor works at low speed, but generates enough pressure for the air to overcome high-density filters of any type. This, in turn, creates two types of noises: Whistling air and vibrations due to the power of the motor:
- The first case (whistling) is avoided by setting the motor through electronics to an ultra-low speed, but with enough pressure to make the air pass through all the filters without making noise. This favors the removal of nanoparticles but requires much more usage time, which is why the equipment operates continuously.
- Vibrations are created by centrifugal forces of the motor and the system of tubular blades. These vibrations are unavoidable, but a method for dissipating them against the high density of the steel body of the equipment has been invented. As this was not enough, a vibration dissipator has been added, which consists of a high-density silicon mass, fired at 1200 °C, which, because of its high weight and being attached to the body of the equipment, absorbs vibrations, preventing noises and nuisances for the user.

The arrangement of these motors and air moving systems must be in the horizontal position and at a specific height from the floor to assure that the best air in the room, i.e. the air that was just treated, reaches the lungs of the patient. However, this must be done without creating any nuisance or interrupting rest; therefore, at the air outlet, there is a framework of steel sheets which carry out two functions:
1) Atomizing the air such that the user does not perceive a bothersome current while he/she sleeps.
2) Heating the steel sheets assuring that the treated air additionally has the desired temperature.

All this air will have previously passed through the ultraviolet ray sterilizer and through the plasma electrostimulator. It is all remotely controlled in a completely automatic manner as a result of the WiFi communication system and the nanoparticle laser measurement system which allows immediately being set automatically according to the pollution both inside and outside the room.

### Placement of the equipment and continuous laminar flow deflector:

The equipment furthermore has a shape which means that it is to be located with respect to the height of the head of a patient at a height between 100 and 130 cm from the floor to be able to send the treated air directly to the patient's respiratory stream, creating a gentle sheet of air directed at the face of the user while he/she sleeps, but without causing any type of nuisance or requiring masks of any type. To that end, a continuous laminar flow has been developed which sends atomized air so gently and continuously that it is barely noticed (unlike the turbulent flow of purifiers, which is distributed everywhere and is a nuisance), thus the patient always breathes in the best air that may be in the room (as it exits the machine) and without nuisances of any type. The equipment thus has solid aluminum diffusers assuring the correct air direction, as well as deflectors in the upper part also made of solid aluminum. Furthermore, the equipment has laminar flow redirecting means, particularly a large-sized deflector slat, preferably made of aluminum, which allows diverting the continuous laminar flow towards the face of the patient for optimal breathing.

The equipment in its entirety is conceived for the patient to immediately receive the treated air. For this purpose it has the described shape, arrangement, and operation.

When a patient sleeps, even though the air is clean, the movement of the patient in bed and against the bedding means that the patient breathes in partially contaminated air due to the particles released by the sheets, by his/her own breathing, by that of his/her companion, or even by multiple substances (including mites) that may be in the pillow. The breathing treatment equipment sends a pure laminar air flow which the patient will not notice since it is a low-intensity continuous laminar flow, carries any type of harmful substance away from the patient's breathing range, and cleans the surface of the patient's bed constantly.

The equipment provides considerable improvements to the quality of sleep, since it requires the patient to be truly relaxed for his/her body to optimally heal. To that end, it incorporates a special design especial in the upper part that imitates the sound box of a musical instrument. This allows transforming the vibrations characteristic of the motor into low-intensity white noise which is used in white function, 2 and 3 as an option for the relaxation while the patient sleeps. The combination of this low-intensity white noise together with the air completely treated 24 hours a day means that the rest and relaxation are deep and restorative. The patient may choose from absolute silence or three white noise intensities.

The equipment can be installed in the wall of an enclosure, but it incorporates a standard support system with or without wheels, which allows the patient to place it in the optimal position for the continuous laminar flow to reach his/her lungs while he/she sleeps, as well as to move it from one room to another depending on who needs it more for health issues.

### Light-sensitive cell for night mode activation:

To do all this, the equipment also incorporates a system which detects when the patient is ready to go to sleep (light-sensitive cell), such that the equipment at that time transitions to night mode, assuring its operation in absolute silence. Thus the patient can sleep without any type of nuisance, without any type of apnea mask, without any type of vibration, or noise, or light or nuisance of any type.

### Design:

The breathing treatment equipment is designed for use as a shelf and for complete integration in the wall of a house, such that the equipment is entirely integrated in the home, no longer being an obstacle in the house like conventional purifiers. Furthermore, its design allows any type of objects such as books, television sets, music equipment, decorations, etc., to be deposited on it since they will not experience interference or vibrations.

The special shape of the equipment is particularly designed to output a film of treated air by means of a continuous laminar flow towards the face of the patient while he/she sleeps such that this said patient always enjoys the best treated air in the room. This shape is unique on the market and vital to the purpose of the present invention. Furthermore, the equipment has a large-sized deflector slat which allows diverting the continuous laminar flow towards the face of the patient for optimal breathing.

It also has a support for use by athletes for the purpose of improving their performance while they train on a treadmill, exercise bike, or other forms of indoor training. Its use with support assures the correct usage height and orientation of the continuous laminar flow towards the patient. The equipment in its entirety is conceived for the patient to immediately receive the treated air. For this purpose it has the described shape, arrangement, and operation.

### Use:

The use of the breathing treatment equipment is entirely different from that of a purifier, since it is not designed for isolated use, but rather for permanent use 24 hours a day. Although a normal purifier also removes fine particles, it is especially designed for isolated uses, giving priority to power or speed to rapidly remove allergens from the environment when necessary. The breathing treatment equipment according to the present invention is designed to be used permanently, working at a very low speed to increase the Brownian effect and thus particularly remove the finest nanoparticles.

This is a continuous low-speed operating process which allows the room in which the equipment is installed to always have an optimal nanoparticle count, and which furthermore directs the treated air towards the respiratory tract of the patient. Particularly during nighttime use, the equipment enhances rest as a result of the insertion of low-intensity white noise. This deep rest is key to the use of the equipment, since only in that state will the recovery and drainage of the patient's entire body be promoted. For that effect and taking into account the fact that the ideal thermal sensation for rest is between 18 and 24 °C, the equipment sends a fresh laminar air flow towards the user, as well as a hot air flow if needed since it incorporates a thermostat and a heat or cool air system.

### Removal of molds and fungi:

The equipment is also used to remove molds and fungi from the home, since it combines the bactericidal task with the thermal function of preventing the home from dropping below 16 %C. It thus prevents dampness and the proliferation of spores. Conventional purifiers are designed for the respiratory system and the equipment is furthermore designed for the circulatory system, relaxation and rest.

### Materials:

Conventional purifiers are basically made of plastic, and after a short usage time turn yellow and age. The breathing treatment equipment comprises materials such as steel, aluminum and silicon, which are designed to operate for up to 50 years continuously, 24 hours a day. The equipment is for professional, medically prescribed use. Furthermore, in order to operate with those powerful, low-speed but high-pressure, low-consumption, steel motors, the equipment must incorporate a system for preventing vibrations. This is achieved as a result of the high-density silicon stabilizer designed to absorb vibrations and convert them into low-intensity white noise. Purifiers incorporate none of this.

### Longevity:

Conventional air purifiers have a short service life, tend to deteriorate, make noise, turn yellow, age, and end up being an environmental problem, as they have to be recycled at a huge economic and ecological cost. The equipment does not age; all its components are interchangeable and 100 % recyclable. The equipment has a service life of 50 years, with all that entails from the ecological point of view and from the user's point of view.

### Arrangement in the home:

The equipment is designed to be placed at a height between 100 and 130 cm from the floor. In an alternative embodiment, the equipment also has wheel systems which allow it to be positioned at that height. It is thereby assured that the continuous laminar flow current reaches the user at the height of his/her face while he/she sleeps, facilitating breathing and preventing the entry of particles that may cause irritations in the airways, making rest difficult. The equipment is designed to be strategically placed in the home to assure a stratum of air in perfect state, even in homes with high ceilings. They are also designed to create laminar flow currents running through the home at the height of the user such that the equipment of the present invention operates by communicating areas with one another, making the air flow from room to room. The equipment therefore has not only a local, isolated function, but rather when strategically placed by professionals, it assures treatment in the entire home for all the people living in it. That is, it is designed for a coordinated use.

### Sound warning system:

The equipment incorporates a sound warning system, particularly an alarm, in the event that pollution levels in the air increase at an alarming rate, which could represent a small fire while the person is sleeping or absent from the room, for example. This alarm will communicate via sound in the room and over the Internet to users authorized by the patient.

### Data acquisition system:

The equipment has a global data acquisition system which allows, by means of the use of artificial intelligence, being autopredictive, thus optimally adapting to the uses and customs of each patient, each home, and each town. All this data, subject to authorization by the client, allows emitting any type of statistic related to treatments, the lowering of medicines, including the curtailment of diseases, and of course control in the longevity of the users of the equipment of the present invention, such that, the improvements in the quality and longevity of the life of patients can be unquestionably documented in the future. Additionally, the equipment incorporates a high-quality display which permanently reports the blood pollution risk level (instantaneous nanoparticle level), as well as the temperature of the room, the systems of the equipment in operation, a light-sensitive control system to adapt to the rest mode, etc.

Therefore, the equipment stands out because of its continuous ultra silent operation, the vibration absorption system for making it silent, the automatic and remote control system for medical monitoring. The system comprises maximum design strength (no plastics) to last for 50 years of continuous use; the equipment is arranged horizontally; and it is a low-consumption system since the equipment operates from only a consumption of 11W/h, assuring an insignificant electricity consumption. Furthermore, the white noise production system is designed for the relaxation, concentration and rest of the user.

Therefore, in a first aspect, the present invention relates to a breathing treatment device for therapeutic purposes for placement thereof in a patient resting enclosure, the device comprising a motor-powered air recirculation system, a set of air purification filters, an ultraviolet ray sterilizer and a plasma electrostimulator. The device is characterized in that it comprises laminar air flow ejection means, laminar air flow redirecting means for redirecting flow towards the head of the patient, a stabilizer and anchoring means to anchor the device to a wall.

In a preferred embodiment, the device is arranged at a height between 100 and 130 cm with respect to the floor of the enclosure. Additionally, the laminar air flow redirecting means for redirecting flow towards the face of the patient comprise an aluminum deflector.

In a preferred embodiment, the device comprises a diffuser interposed between the patient and the laminar flow. In another preferred embodiment, the device comprises a support system for arranging the device at a height between 100 and 130 cm with respect to the floor of the enclosure, preferably with wheels.

In a preferred embodiment, the device comprises wireless transmission means, preferably WiFi and/or Bluetooth and/or a LED display (130).

In a preferred embodiment, the device comprises a pre-filter, a blue antibacterial filter, a HEPA 13 filter and an activated carbon filter with catalytic platinum (124). Furthermore, in another preferred embodiment, the device comprises a stainless steel filter (125). In a preferred embodiment, the stabilizer comprises silicon and/or steel.

In a preferred embodiment, the device comprises a silent operating mode or a low-intensity white noise operating mode. Furthermore, in another preferred embodiment, the device comprises a multilevel nanoparticle laser measuring device and/or an audible alarm.

In another aspect, the invention relates to a method for the treatment of breathing conditions. The method comprises placement of the device according to the preceding claims at a height between 100 and 130 cm with respect to the floor of an enclosure and the application of a laminar air flow generated by said device above the head of a patient.

### Description of the drawings

As a complement to the description being provided, and for the purpose of helping to make the features of the electric generator more readily understandable, in accordance with a practical preferred embodiment thereof, said description is accompanied by a set of drawings which, by way of illustration and not limitation, represent the following:
Figure 1 shows the breathing treatment equipment according to the present invention and the set of filters forming it.
Figure 2 shows the LED display of the breathing treatment equipment according to the present invention.
Figure 3 shows the breathing treatment equipment according to the present invention with a support system with wheels.
Figure 4 shows the breathing treatment equipment according to the present invention with a diffuser at the outlet of the air current.
Figure 5 shows the breathing treatment equipment according to the present invention in operation next to a patient.

### Preferred embodiment of the invention

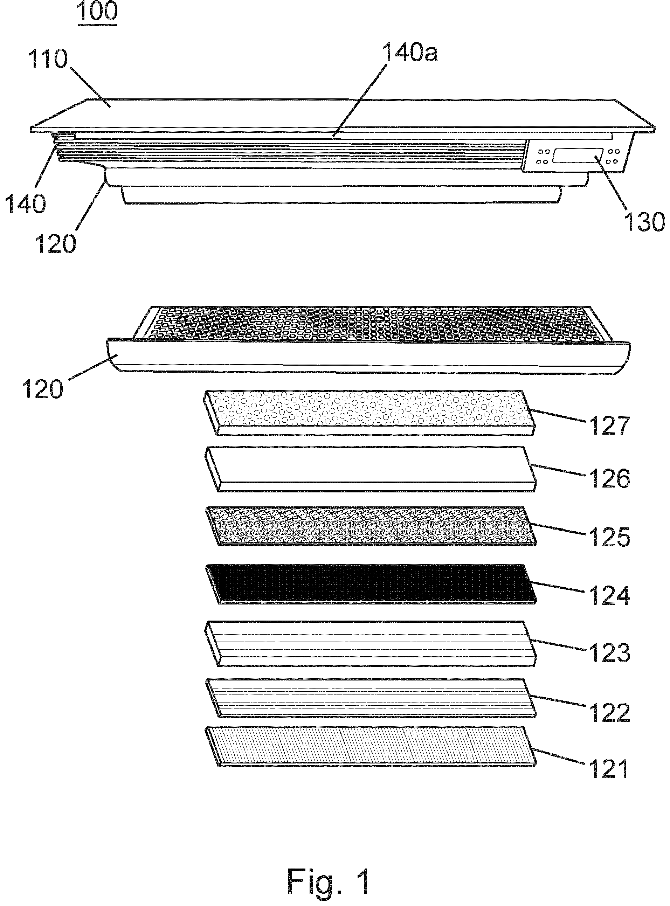

The breathing treatment equipment (100) works in a continuous (24 hours) and ultra silent manner to aid in the rest and regeneration of the body while a patient sleeps. A casing made of steel, as well as a front area comprising laminar air flow ejection means (140) and a LED display (130) can be observed in Figure 1. The breathing treatment equipment (100) outputs a treated laminar air flow which can be diverted by means of laminar air flow redirecting means for redirecting flow towards the face of the patient, comprising a longitudinal aluminum deflector slat (140a).

The breathing treatment equipment (100) comprises a filter system arranged in the structure (120):
To breathe in the treated air, the air is first passed through a washable pre-filter (121) removing the coarsest particles to prolong the life of the filter. Then the air is passed through a first sterilizing antibacterial filter (122). The air continues through a high-density and large-sized HEPA 13 filter (123), thus removing 99.9 % of the suspended particles.

The air is again filtered through an activated carbon filter (124) comprising activated carbon capsules catalyzed by platinum, thus removing gases, smells and volatile organic compounds VOCs, and HVOC formaldehydes.

That air that is so filtered is passed through a photocatalytic (UV) filter (126) which destroys bacteria, viruses, volatile organic compounds and nitrogen dioxide, thus achieving sterilized air.

If there is still any particle of less than 0.1 microns (0.0001 mm), the filter system comprises a double plasma electrostimulation filter (127) which ionizes the air such that the particles become electrically charged, adhering to one another, creating larger particles, which will be filtered again.

Furthermore the equipment (100) comprises a high-density steel and/or silicon stabilizer (110) which absorbs any type of vibrations, along with the body of the equipment which has been made 100 % from steel and silicon for such function, thus creating a high-density module which, when tightly screwed to the equipment, provides rigidity, eliminates vibrations and allows giving the equipment a service life of more than 35 years.

Furthermore, the equipment comprises a stainless steel filter (125); particularly, the stainless steel filter (125) comprises a polished stainless steel grating which allows using the equipment (100) while the removed particles are extracted from the filters to clean them. The stainless steel filter (125) comprises at least three functions:
1- It prevents access to moving parts of the equipment (100) to enable using same without the filter package which can be changed during the extraction and cleaning of said filter package. This allows increasing the air flow rate of the equipment (100) and decreasing the sound level. It may be useful at times when the room is clean and a greater capacity for refreshing or heating the environment is needed.
2.- The stainless steel filter (125) allows coarse material filtration regardless of the filtration performed by the filter package.
3.- The stainless steel filter (125) generates ultraviolet ray reflections, which causes these rays to reach all the internal parts of the equipment, including the blades for circulating the flow rate, the carbon filter and other internal parts. These ultraviolet light reflections favor internal sterilization of the entire equipment (100). This allows never sending bacteria out towards users.

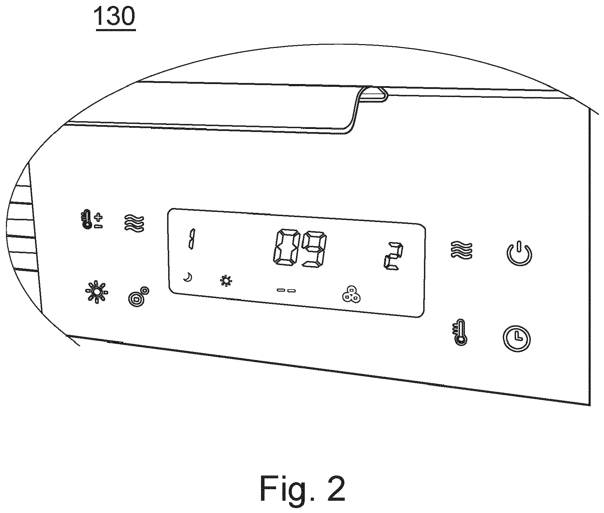

The high-quality LED display (130) which permanently reports the blood pollution risk level (instantaneous nanoparticle level), as well as the temperature of the room, the systems of the equipment in operation, a light-sensitive control system to adapt to the rest mode, etc., can be seen in Figure 2.

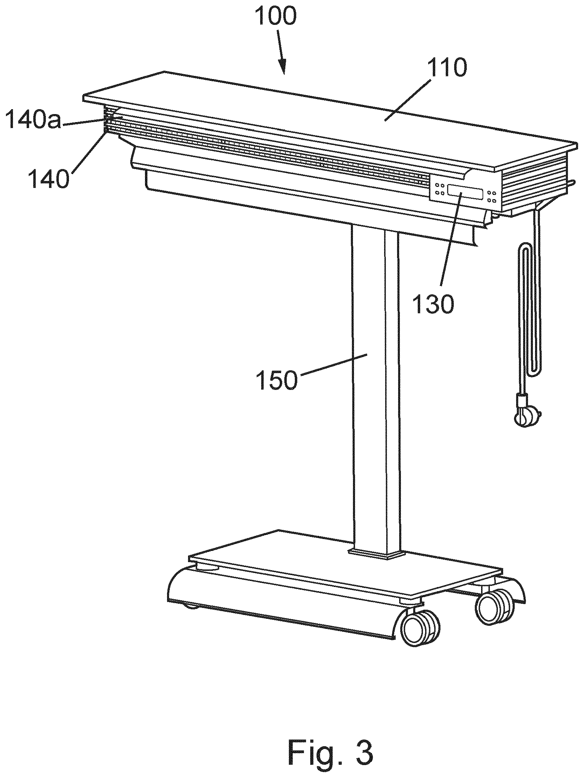

The breathing treatment equipment (100) according to the present invention with a support system with wheels (150) which allows placing it in the optimal position for the continuous laminar flow to reach the lungs of the patient while he/she sleeps, particularly at a height between 100 and 130 cm with respect to the floor of the enclosure, as well as moving it from one room to another depending on who needs it the most for health issues, can be seen in Figure 3. In a preferred embodiment, the filter comprises anchoring means which allow screwing the equipment (100) to the wall, rendering the support system with wheels (150) dispensable. The laminar air flow ejection means (140) and the longitudinal aluminum deflector slat (140a) for redirecting the laminar flow towards the face of the patient can also be seen.

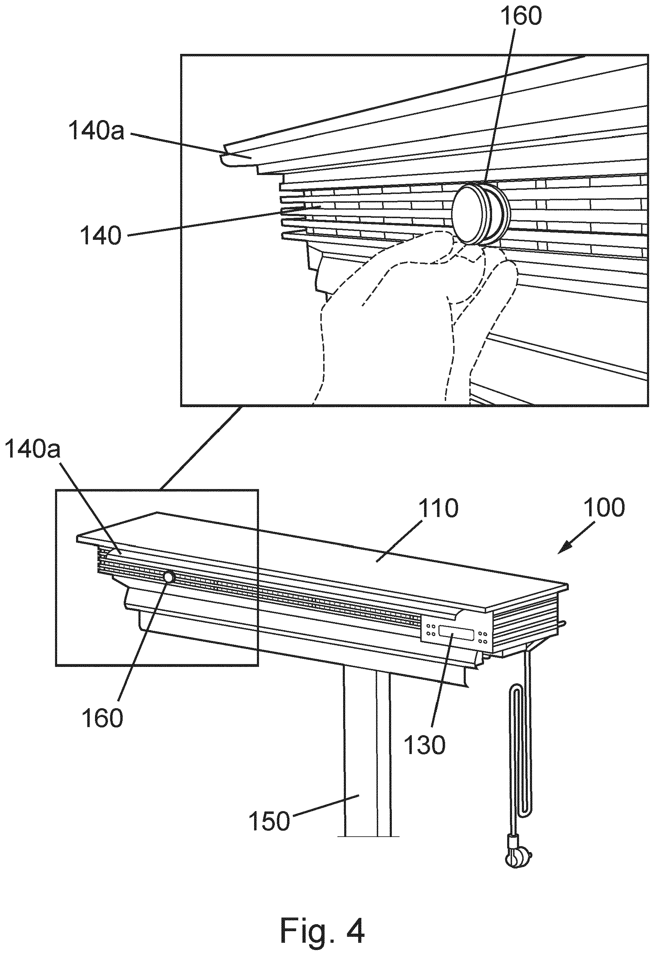

Figure 4 shows the breathing treatment equipment (100) according to the present invention with a diffuser (160) at the outlet of the air current. The breathing treatment equipment (100) incorporates a diffuser at the outlet of the treated air current which allows the application of different substances via inhalation. The diffuser (160) allows incorporating different medically prescribed treatments, such as bronchodilators or substances of another type which enhance the efficiency of the breathing treatment and even allow intensive isolated uses to treat different kinds of attacks (asthma attacks, allergy attacks, etc.).

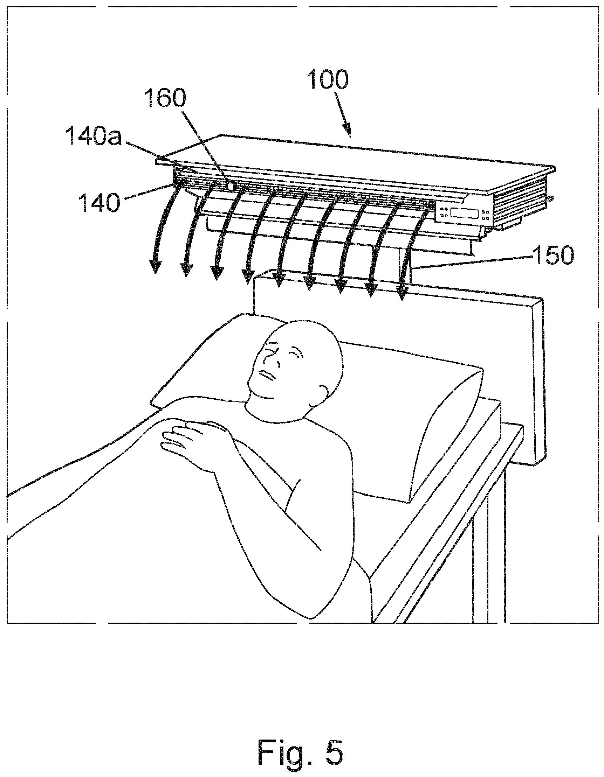

Figure 5 shows the breathing treatment equipment (100) according to the present invention together with the diffuser (160) at the outlet of the air current above the bed of a patient. The breathing treatment equipment (100) comprises the support system (150) for arranging the device at a height between 100 and 130 cm with respect to the floor of the enclosure, which allows the ejection of a laminar air flow through means (140) to be redirected by means of the aluminum deflector (140a) towards the face of the patient and thus achieving optimal breathing.

Although reference has been made to specific embodiments of the invention, the breathing treatment units described herein are susceptible to a number of variations and modifications, and all the mentioned features can be replaced with other technically equivalent features without departing from the scope of protection defined by the claims.

## Claims

1. Breathing treatment device (100) for therapeutic purposes for placement thereof in a patient resting enclosure, the device comprising a motor-powered air recirculation system, a set of air purification filters (121, 122, 123, 124), an ultraviolet ray sterilizer (126) and a plasma electrostimulator (127), the device **characterized in that** it comprises:
- laminar airflow ejection means (140);
- laminar air flow redirecting means (140a) redirecting flow to the head of the patient;
- a stabilizer (110); and
- anchoring means to anchor the device (100) to a wall.

2. Breathing treatment device (100) according to claim 1, wherein the breathing treatment device (100) is arranged at a height between 100 and 130 cm with respect to the floor of the enclosure.

3. Breathing treatment device (100) according to the preceding claims, wherein the laminar air flow redirecting means (140a) redirecting flow to the face of the patient comprise an aluminum deflector.

4. Breathing treatment device (100) according to the preceding claims, further comprising a diffuser (160) interposed between the patient and the laminar flow.

5. Breathing treatment device (100) according to the preceding claims, further comprising a support system (150) for arranging the device at a height between 100 and 130 cm with respect to the floor of the enclosure, preferably with wheels.

6. Breathing treatment device (100) according to the preceding claims, further comprising wireless transmission means, preferably WiFi and/or Bluetooth.

7. Breathing treatment device (100) according to the preceding claims, further comprising a LED display (130).

8. Breathing treatment device (100) according to the preceding claims, wherein the set of air purification filters comprises a pre-filter (121), a blue antibacterial filter (122), a HEPA 13 (High Efficiency Particulate Air) filter (123), an activated carbon filter with catalytic platinum (124).

9. Breathing treatment device (100) according to the preceding claims, further comprising a stainless steel filter (125).

10. Breathing treatment device (100) according to the preceding claims, wherein the stabilizer (110) comprises silicon and/or steel.

11. Breathing treatment device (100) according to the preceding claims, comprising a silent operating mode or a low-intensity white noise operating mode.

12. Breathing treatment device (100) according to the preceding claims, further comprising a multilevel nanoparticle laser measuring device.

13. Breathing treatment device (100) according to the preceding claims, further comprising an audible alarm.

14. Method for the treatment of breathing conditions, the method comprising:
- placement of the device according to the preceding claims at a height between 100 and 130 cm with respect to the floor of a patient resting enclosure; and
- application of a laminar air flow generated by said device on the head of the patient.
